# EUROPEAN PATENT SPECIFICATION

(11) **EP 2 900 272 B1**
(45) Date of publication and mention of the grant of the patent: **06.06.2018**
(21) Application number: 13840242.5
(22) Date of filing: 30.09.2013
(51) Int. Cl.: A61K 47/36, A61K 31/485, A61K 9/00, A61K 36/888, A61K 47/26, A61K 9/20, A61K 31/135, A61K 31/137, A61K 31/4468, A61K 31/451, A61K 31/5513, A61P 25/04, A61P 25/24

(54) **ABUSE DETERRENT PHARMACEUTICAL COMPOSITION COMPRISING KONJAC GLUCOMANNAN**
MISSBRAUCHSSICHERE PHARMAZEUTISCHE ZUSAMMENSETZUNG MIT KONJAC-GLUCOMANNAN
COMPOSITION PHARMACEUTIQUE DE DISSUASION D'ABUS COMPRENANT DU KONJAC GLUCOMANNAN

(30) Priority: 28.09.2012 CA 2791206
(43) Date of publication of application: 05.08.2015
(73) Proprietor: Pharmascience Inc., Montréal, Québec H4P 2T4 (CA)
(72) Inventor: BHANDARI, Krishna Hari, Edmonton, Alberta T6X 0N2 (CA); TALWAR, Naresh, Kirkland, Quebec H9H 5L1 (CA)
(74) Representative: HGF Limited
(86) International application number: PCT/CA2013/000835
(87) International publication number: WO 2014/047731

(56) References cited:
- CA-A1- 2 152 795
- CA-A1- 2 674 536
- CA-A1- 2 707 980
- US-A1- 2008 152 595
- FAN J ET AL: "In vitro evaluations of konjac glucomannan and xanthan gum mixture as the sustained release material of matrix tablet", CARBOHYDRATE POLYMERS, APPLIED SCIENCE PUBLISHERS, LTD. BARKING, GB, vol. 73, no. 2, 19 July 2008 (2008-07-19), pages 241-247, XP022589712, ISSN: 0144-8617, DOI: 10.1016/J.CARBPOL.2007.11.027 [retrieved on 2007-11-23]
- ALONSO-SANDE M ET AL: "Glucomannan, a promising polysaccharide for biopharmaceutical purposes", EUROPEAN JOURNAL OF PHARMACEUTICS AND BIOPHARMACEUTICS, ELSEVIER SCIENCE PUBLISHERS B.V., AMSTERDAM, NL, vol. 72, no. 2, 1 June 2009 (2009-06-01), pages 453-462, XP026119131, ISSN: 0939-6411, DOI: 10.1016/J.EJPB.2008.02.005 [retrieved on 2008-02-16]
- EDWIN R MORRIS ET AL: "Gelation of gellan A review", FOOD HYDROCOLLOIDS, ELSEVIER BV, NL, vol. 28, no. 2, 3 January 2012 (2012-01-03), pages 373-411, XP028467351, ISSN: 0268-005X, DOI: 10.1016/J.FOODHYD.2012.01.004 [retrieved on 2012-01-16]

## Description

### FIELD OF THE INVENTION

The invention relates to pharmaceutical dosage forms for oral administration which are developed to provide a deterrent to potential abusers. More specifically, this invention relates to abuse-deterrent modified release pharmaceutical dosage forms containing drugs prone to abuse.

### BACKGROUND OF THE INVENTION

One of the most widespread health issues affecting society at the present time is pain. Pain can come from different diseases and/or conditions but to those affected by it, there is often only one solution and it is the treatment of the symptoms. Through the use of medication pain can be alleviated, controlled and/or diminished to allow individuals afflicted with pain to lead otherwise normal and fulfilling lives. However, because of the effectiveness of some medication to control, alleviate and/or diminish pain (or its symptoms) there is widespread abuse of such medication. These medications can also be abused to get euphoric "highs" or other mood elevating effects by abusers. This explains the reason why pain medication commands a relatively high price on the black market.

Because of the necessity of the availability of pain medication and the potential for abuse, there have been a number of developments in abuse resistant or tamper deterrent technology to have such medication remain widely available without being an easy source of abuse.

To this effect, a variety of alternative technologies have emerged to curb potential abuse by targeting different aspects of extraction of the drug from the commercially available pain medication. To wit, there has been the development of technologies which prevent the extraction of drug for intranasal administration, parenteral administration.

Abuse is an ongoing concern that many pharmaceutical companies have tried to address. Since abuse is prevalent towards opioid drugs such as oxycodone, hydromorphone and the like, most of the tamper resistant formulations are directed to these types of drugs.

Abusers are always looking for extended release dosage forms due to the presence of higher amount of drug per unit of the dosage form. In spite of all of the various developments in abuse-deterrent formulations, there still exists a need to develop an abuse-deterrent formulation which can prevent abuse by preventing injection, inhalation, and/or oral abuse of a non-abuse-deterrent formulation by decreasing the "availability" of the active pharmaceutical ingredient to a potential abuser.

One method to prevent abuse of pharmaceutical formulation is to include a bittering agent which is meant to deter an abuser from tampering with a dosage form. Typically, tampering of a dosage form would allow an abuser to inhale or swallow the API recovered from the tampered dosage form. A bittering agent is included to be released when one tampers with a dosage form, the latter thus imparting an unpleasant taste to the API to the abuser upon inhalation and/or swallowing of the tampered dosage form.

Another method of providing a tamper resistant tablet is to produce one that can resist milling or crushing thereby preventing the step prior to an extraction by solvent. Purdue has developed and commercialized a formulation of controlled release oxycodone which has such physical characteristics.

Collegium Pharmaceutical, Inc./Endo Pharmaceuticals, Inc. have also developed an oxycodone-containing formulation which is meant to deter tampering through chewing, milling, inhalation and intravenous injection. Such tamper deterrent characteristics is obtained in this case through the use a of micro-particle formulation.

Yet another company that has developed a tamper deterrent oxycodone formulation is IntelliPharmaCeutics Ltd. They have also developed a technology which is meant to make it very difficult to chew, mill, or inject the oxycodone formulation. It is said that the formulation does not dose dump, which means that even through abuse, the formulation will not release all of the oxycodone immediately.

King Pharmaceuticals has approached the tamper deterrent of oxycodone formulation in a different manner through the use of a liquid matrix which is expected to prevent dumping through exposure to one of a variety of mechanical abuse (such as crushing, milling, chewing) or exposure of the formulation to solvents.

Another method to deter abuse of pharmaceutical formulation is to include a gelling agent which is intended to make it much more difficult for an abuser to tamper the dosage form and subsequently inhale, inject, and/or swallow the API recovered from the tampered dosage form.

In order to prevent one from ingesting a tampered dosage form, one method used is to prevent such tampering by including a gelling agent which will prevent the release of the drug when one attempts extraction through the use of solvents.

Essentially, a gelling agent works when a dosage form is being dissolved for extraction of the drug by forming a gel when placed in a solvent. Once formed, the gel prevents the misuse of the drug because of the gel formation which, in turn, cannot be abused intranasally or intravenously.

Abusers may attempt to inhale through nasal inhalation a tampered dosage form. Some tamper deterrent dosage form have taken this route of abuse into account and have developed dosage form containing a gelling agent which becomes a gel upon inhalation into the nose. The moisture of the mucous membranes in the nose leads to the formation of a gel. The formation of such a gel in the nasal cavity generally leads to some local irritation thereby making such abuse less desirable from an abuser's point of view. Furthermore, the main idea is to stop the passage of the drug into the lung for better absorption. Also, the gel might block the nasal passage and a reflux action will help to throughout the gelled sample. In addition, the gelled matrix could release the drug in an extended release pattern.

Gels have been used in the preparation of tamper deterrent formulations, see for example Oxycodone (e.g., Oxecta™ by Acura Pharmaceuticals, commercialised by Pfizer). However, this dosage form is an immediate release dosage form.

Tramadol and morphine are two other opioids for which tamper deterrent dosage forms have been developed and are undergoing studies to assess the impact on deterrence of those technologies.

Konjac is a plant containing a number of insoluble material including a linear polysaccharide containing both mannose and glucose, konjac glucomannan. Konjac glucomannan is extracted from the tubers of the *Amorphophallus Konjac,* it is found in Asia, mostly from Japan and China down to Indonesia.

Konjac glucomannan is generally recognized as safe (GRAS), it has been approved as a food ingredient in Europe since the late 1990's. It has been used extensively in the food industry in breads, frozen products, pastas, dressings, and various types of beverages for such purposes as: binding, increasing suspension and viscosity, adding texture and moisture retention. Konjac forms a gel that is temperature stable when it is placed in the presence of an alkaline coagulant such as sodium carbonate. Konjac glucomannan gel is used most extensively in Japanese foods. The use of konjac flour provides a certain amount of dietary fiber which provides demonstrable results in cholesterol and weight reduction.

It is thought that the gelation of konjac glucomannan is driven by the deacetylation by alkali. The mechanism for this has been studied but not yet elucidated. Gellation studies of konjac glucomannan gel seem to imply that a deacetylation reaction results in the aggregation of the modified molecules, which have reduced flexibility.

Canadian Patent No. 2,372,649 discloses the use of konjac glucomannan to stabilize or impart some texture to some food items. It is suggested that it can act as a fat substitute and be used in foods such as peanut butter, margarine, frozen desserts and other.

Canadian Patent No. 2,180,334 discloses the use of konjac glucomannan in coprecipitate form with a galactomannan to form the base of gelled or thickened food items such as food spread, salad dressings, cheese spreads, mayonnaise and cosmetic or pharmaceutical liquids, creams or lotions.

Canadian Patent Application 2,733,231 discloses the use of polysaccharide gums in hydrophilic matrix carriers for the sustained delivery of drugs of varying ranges of solubility. The application mentions various gums including konjac, but provides no examples of the use of this gum in making sustained release formulations. It emphasizes the use of guar gum in combination with mannitol.

US Patent Application No. 2007/0128285 discloses the use of konjac to prepare a gellied pharmaceutical preparation for the administration of 5HT₃-receptor antagonist. There is no teaching of modified release in this application. Canadian Patent No. 2,152,795 discloses the use of konjac glucomannan as a sustained release excipient. There are provided some examples with theophylline as active ingredient. Canadian patent 2 674 536 discloses abuse resistant polyglycol-based pharmaceutical compositions. Glucomannan is mentioned as a further suitable polymer. The exemplified compositions provide for sustained release and show resistance to abuse in a crush test, melting test and extraction test. Some more references are known from published articles which discusses physical properties of konjac glucomannan as follows: Liu J - Preparation of konjac glucomannan-based pulsatile capsule for colonic drug delivery system and its evaluation in vitro and in vivo, Carbohydrate Polymers 87 (2012), pp. 377-382; Alvarez-Mancenido F - Konjac glucomannan and konjac glucomannn/xantham gum mixtures as excipients for controlled drug delivery systems, Int. Journal of Pharmaceutics, 349 (2008), pp.11-18; Xu X - Characterization of konjac glucomannan-gellan gum blend films and their suitability for release of nisin incorporated therein, Carbohydrate Polymers, 70 (2007), pp. 192-197; Wang K - Alginate-konjac glucomannan-chitosan beads as controlled release matrix, Int. Journal of Pharmaceutics, 244 (2002), pp. 117-126; Cheng LH - Effects of acid modification on physical properties of konjac glucomannan (KGM) films, Food Chemistry, 103 (2007), pp. 994-1002; Wang C - Synthesis of the KMB-Drug Delivery Carrier, Physics Proceedings, 33 (2012), pp. 20-24; Ji Y - In vitro evaluation of Konjac glucomannan as novel excipients for floating systems, Journal of Controlled Release, 152 (2011), pp. e34-e36; Rana V - Modified gums: Approaches and applications in drug delivery, Carbohydrate Polymers, 83 (2011), pp. 1031-1047; Liu Z - Polysaccharides-based nanoparticules as drug delivery systems, Advanced Drug Delivery Reviews, 60 (2008), pp. 1650-1652; Wu C - Structural characterization and properties of konjac glucomannan/curdlan blend films, Carbohydrate Polymers, 89 (2012), pp. 497-512; Yu H - Synthesis and properties of novel hydrogels from oxidized konjac glucomannnan crosslinked gelatin for in vitro drug delivery, Carbohydrate Polymers, 72 (2008), pp. 479-489; Alonso-Sande M - Glucomannan, a promising polysaccharide for biopharmaceutical purposes, Eur. Jour. Of Pharmaceutics and Biopharmaceutics, 72 (2009), pp. 453-462; Wen, X - Preparation and characterization of konjac glucomannan-poly(acrylic acid) IPN hydrogels for controlled release, Carbohydrate Polymers, 78 (2009), pp. 193-198.

A review of the teachings of these articles reveals that while there are discussions on the properties of konjac glucomannan as a promising excipient for modified release compositions, there is no teaching of the use of konjac glucomannan in an abuse deterrent pharmaceutical composition.

In light of the documents described and discussed it is clear that there is still a need to develop a technology that can be applied to pharmaceutical compositions to curb abuse by drug abusers whether they are recreational abusers or individuals involved in the illicit drug trade. The inventors have developed a modified release and abuse deterrent technology for use in pharmaceuticals using a known food excipient (or additive). The inventors have applied this food excipient (or additive) to a technology which positively increases the difficulty of extracting the therapeutic drug present in a pharmaceutical dosage form.

### SUMMARY OF THE INVENTION

A first aspect of the invention provides a modified release orally administrable abuse-deterrent pharmaceutical composition comprising: a therapeutically effective amount of an active pharmaceutical ingredient, konjac glucomannan and gellan gum.

A second aspect of the invention provides a modified release orally administrable abuse-deterrent pharmaceutical composition according to claim 8.

According to one aspect of the present invention, there is provided a modified release orally administrable abuse-deterrent pharmaceutical composition according to claim 12.

A further aspect of the invention provides the modified release orally administrable abuse-deterrent pharmaceutical composition of the invention for use in the treatment of pain or depression.

Another aspect of the invention relates to a modified release orally administrable abuse-deterrent pharmaceutical composition for use in the treatment of pain, depression, anxiety or sleep disorders, narcolepsy and Attention-Deficit/Hyperactivity Disorder (ADHD) in human, wherein said composition comprises: a therapeutically effective amount of an active pharmaceutical ingredient susceptible to abuse; konjac glucomannan; gellan gum; optionally, at least one nasal irritant; and at least one other pharmaceutically acceptable excipient.

The disclosure provides a pharmaceutical dosage form for oral administration which has the potential of being abuse deterrent and thus be less likely for parenteral abuse than other dosage forms.

The disclosure provides a pharmaceutical dosage form for oral administration which has the potential of being abuse deterrent and thus be less likely for oral and/or nasal abuse than other dosage forms.

Preferably, the active pharmaceutical ingredient is selected from the group consisting of: opioids and morphine derivatives; antidepressants; stimulants; and other drugs. Preferably, the opioids and morphine derivatives are selected from the group consisting of: oxycodone HCI, hydrocodone bitartrate hydromorphone, oxymorphone, meperidine, propoxyphene, fentanyl & analogs, tramadol, codeine, morphine and methadone. Preferably, the antidepressants are selected from the group consisting of: barbiturates; benzodiazepines; and sleep medications. Preferably, the stimulants are selected from the group consisting of: amphetamines and methylphenidate. Preferably, the other drugs comprise dextrometorphan.

Preferably, the composition according to the present invention provides release of the active pharmaceutical ingredient over at least 8 hours, preferably over at least 12 hours and more preferably over 24 hours.

According to one aspect of the present invention, there is provided a use of konjac glucomannan in the manufacture of a modified release orally administrable abuse-deterrent pharmaceutical composition for the treatment of pain, said composition comprising a therapeutically effective amount of an active pharmaceutical ingredient admixed with konjac glucomannan and at least one other pharmaceutically acceptable excipient.

According to another aspect of the present invention, there is provided a use of konjac glucomannan in the manufacture of a modified release orally administrable abuse-deterrent pharmaceutical composition for the treatment of depression, said composition comprising a therapeutically effective amount of an active pharmaceutical ingredient admixed with konjac glucomannan and at least one other pharmaceutically acceptable excipient.

Preferably, konjac glucomannan is present in an amount ranging from 3% to 90 % w/w, more preferably from 10% to 80% w/w, even more preferably from 25% to 65% w/w, even more preferably from 30 % to 60 % w/w, most preferably from 30% to 50% w/w.

The disclosure provides a modified release orally administrable abuse-deterrent pharmaceutical composition comprising: a therapeutically effective amount of an active pharmaceutical ingredient, konjac glucomannan and at least one gelling polymeric compound, wherein said composition becomes an uninjectable and unsyringeable gel when tampered and exposed to aqueous, alcoholic, acidic or basic media.

Preferably, the at least one gelling polymeric compound is selected from the group consisting of: gellan gum, xanthan gum, carrageenan, carbopol, polyethylene oxide, hydroxypropyl methylcellulose (HPMC), and combination thereof.

The disclosure also provides a modified release orally administrable abuse-deterrent pharmaceutical composition comprising: at least one pharmaceutically active ingredient susceptible to abuse; konjac glucomannan; at least one other gelling polymeric compound selected from the group consisting of: gellan gum, xanthan gum, polyethylene oxide, carrageenan, carbopol, hydroxypropylmethylcellulose and combinations thereof; optionally, at least one nasal irritant selected from the group consisting of: sodium lauryl sulfate, capsaicin and capsaicin analogs selected from the group consisting of resiniferatoxin, tinyatoxin, heptanoylisobutylamide, heptanoyl guaiacylamide, other isobutylamides or guaiacylamides, dihydrocapsaicin, homovanillyl octylester, nonanoyl vanillylamide, and mixtures thereof; and at least one other pharmaceutically acceptable excipient, wherein said formulation provides release of the active pharmaceutical ingredient and has an *in vitro* dissolution profile where not more than 60 % of the pharmaceutically active ingredient is dissolved in 6 hours after administration as determined by USP paddles method described in USP XXVI (2003). Preferably, the third gelling polymeric compound, is present in an amount ranging from 1.0% to 30% w/w.

By modified release, the inventors refer to compositions which behave differently from immediate release composition. In a preferred embodiment of the present invention, modified release refers to sustained release where the active pharmaceutical ingredient is released at a predetermined rate over an extended period of time, i.e. up to 8 hours, up to 12 hours, up to 24 hours.

In a preferred embodiment of the present invention, modified release refers to delayed or extended release where the active pharmaceutical ingredient is released with a delay after ingestion.

In a preferred embodiment of the present invention, modified release refers to controlled release where the active pharmaceutical ingredient is released constant over an extended period of time after ingestion.

In a preferred embodiment of the present invention, modified release refers to a combination of delayed and sustained release where the active pharmaceutical ingredient is released with a delay after ingestion at a predetermined rate and over an extended period of time.

According to one aspect of the present invention, the gellan gum is present in an amount ranging from about 1.0% to about 30% w/w.

According to one aspect of the present invention, the gelling polymeric compound is xanthan gum and is present in an amount ranging from about 1.0% to about 30% w/w.

According to another aspect of the present invention, the gelling polymeric compound is polyethylene oxide and is present in an amount ranging from about 1.0% to about 30% w/w.

According to yet another aspect of the present invention, the gelling polymeric compound is carrageenan and is present in an amount ranging from about 1.0% to about 30% w/w.

According to yet another aspect of the present invention, the gelling polymeric compound is carbopol and is present in an amount ranging from about 1.0% to about 30% w/w.

According to yet another aspect of the present invention, the gelling polymeric compound is hydroxypropylmethylcellulose and is present in an amount ranging from about 1.0% to about 30% w/w.

According to one aspect of the present invention, the modified release orally administrable abuse-deterrent pharmaceutical composition further comprises a sodium lauryl sulfate, and other nasal irritants selected from the group consisting of: capsaicin and capsaicin analogs, resiniferatoxin, tinyatoxin, heptanoylisobutylamide, heptanoyl guaiacylamide, other isobutylamides or guaiacylamides, dihydrocapsaicin, homovanillyl octylester, nonanoyl vanillylamide, and mixtures thereof. Preferably, is used sodium lauryl sulfate.

The disclosure provides a modified release orally administrable abuse-deterrent pharmaceutical composition comprising: at least one pharmaceutically active ingredient susceptible to abuse; konjac glucomannan; at least one gelling polymeric compound, optionally at least one nasal irritant, and at least one pharmaceutically acceptable excipient, wherein said composition provides a modified release of the active pharmaceutical ingredient susceptible to abuse when the tablet is taken orally and, upon tampering and exposure to an aqueous, alcoholic, acidic and/or basic media, said formulation becomes an uninjectable and unsyringeable gel.

According to yet another aspect of the present invention, there is provided a use of konjac glucomannan in the manufacture of a modified release orally administrable abuse-deterrent pharmaceutical composition for the treatment of pain, or depression, wherein said composition comprising: a therapeutically effective amount of an active pharmaceutical ingredient susceptible to abuse, konjac glucomannan, and, optionally, at least one nasal irritant, preferably sodium lauryl sulphate.

Further aspect of the present invention, there is provided a use of a modified release orally administrable abuse-deterrent pharmaceutical composition for the treatment of pain, depression, anxiety or sleep disorders, narcolepsy and Attention-Deficit/Hyperactivity Disorder (ADHD) in human, wherein said composition comprises: a therapeutically effective amount of an active pharmaceutical ingredient susceptible to abuse; konjac glucomannan; at least one gelling polymeric compound; optionally, at least one nasal irritant; sodium lauryl sulphate and at least one other pharmaceutically acceptable excipient.

Preferably, the modified release orally administrable abuse-deterrent pharmaceutical composition according to the present invention contains konjac glucomannan in an amount ranging from 3% to 90% w/w of the total tablet without coating. More preferably, from 10% to 80% w/w, more preferably still from 25% to 65 % w/w, even more preferably still from 30% to 65 %w/w, and most preferably from 30% to 50 % w/w.

According to a preferred embodiment of the present invention, there is provided a modified release abuse-deterrent orally administrable composition which comprises a nasal irritant to deter abuse via nasal administration. If an abuser crushes the dosage form, the nasal irritant is exposed. The nasal irritant is present to discourage inhalation of the crushed dosage form by inducing pain and/or irritation to the abuser upon nasal administration. The modified release orally administrable abuse-deterrent pharmaceutical composition further comprises a nasal irritant selected from the group consisting of: sodium lauryl sulphate, capsaicin, a capsaicin analogs, resiniferatoxin, tinyatoxin, heptanoylisobutylamide, heptanoyl guaiacylamide, other isobutylamides or guaiacylamides, dihydrocapsaicin, homovanillyl octylester, nonanoyl vanillylamide, and mixtures thereof. More preferably, the nasal irritant is capsaicin, even more preferably is sodium lauryl sulphate and is present in an amount ranging from about 1.0% w/w to about 10% w/w.

### BRIEF DESCRIPTION OF THE DRAWINGS

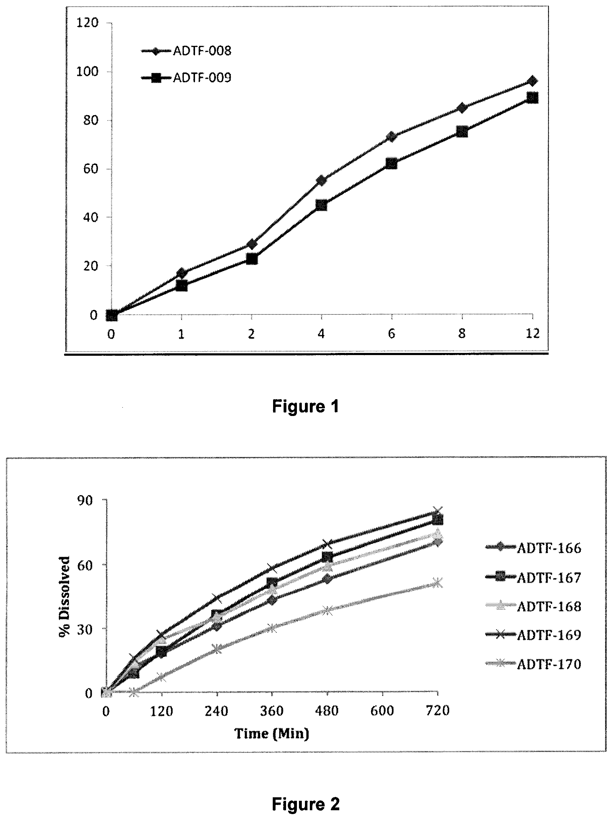

**Figure 1** is a graph showing the representative release of drugs from abuse-deterrent pharmaceutical compositions containing hydromorphone (ADTF-008) and oxycodone(ADTF-009). Percentage drug release from hydromorphone and oxycodone abuse- deterrent extended release tablet mentioned in Example 1 and Example 2 in 900 mL dissolution media (Phosphate buffer pH 6.8) using USP1 dissolution apparatus at 100 rpm is shown. The units of the graph are relative percentage cumulative of drug *vs* time (hours)(Y-axis: % drug release, X-axis: time (h)).
**Figure 2** is a graph showing the representative release of drugs from abuse-deterrent pharmaceutical compositions containing hydromorphone (ADTF-166, ADTF-167, ADTF-168, ADTF-169 and ADTF-170). Percentage drug release from hydromorphone abuse deterrent modified release tablet mentioned in Examples 3, 4, 5, 6 and 7 in 900 mL dissolution media (Phosphate buffer pH 6.8) using USP1 dissolution apparatus at 100 rpm is shown. The units of the graph are relative percentage cumulative of drug *vs* time (hours)(Y-axis: % drug release, X-axis: time (h)).

### DETAILED DESCRIPTION OF THE INVENTION

Before the present compositions and methods of use are disclosed and described, it is to be understood by a person skilled in the art that, unless otherwise indicated, this invention is not limited to specific pharmaceutical carriers, or to particular administration regimens. It is also to be understood that the terminology used herein is for the purpose of describing particular embodiments only and is not intended to be limiting.

The term "tampered dosage form" is defined for purposes of the present invention to mean that the dosage form has been manipulated by mechanical, thermal, and/or chemical means with the intended goal of affecting the original physical integrity and properties of the commercially available dosage form. An example of tampering of a dosage form is when one attempts to extract the therapeutic agent from a commercially available dosage form for immediate release when it is actually formulated into a modified release dosage form. Extraction of a therapeutic agent from a commercially available dosage form can also be done in order to render the therapeutic agent available to abuse by an alternate administration route, e. g., parenterally, nasally or by instillation into nostril.

The tampering can be, e.g., by means of crushing, milling, shearing, grinding, chewing, dissolution in a solvent, heating or even any combination of such acts.

In the present application, the terms or expressions "active agent", "therapeutically active agent", "therapeutic agent", "active ingredient", "active pharmaceutical ingredient", "drug", "pharmacologically active agent", "pharmaceutically acceptable active agent" and/or "pharmaceutically acceptable active substance" are used interchangeably to refer to a chemical material, compound, agent or substance that has measurable specified or selected physiologic activity when administered to a subject in a pharmaceutically significant or effective amount. The active agent can be a therapeutic, a prophylactic, or a diagnostic agent. These terms of art are well-known in the pharmaceutical and medicinal arts.

In accordance with a preferred embodiment of the present invention, there is provided an abuse-deterrent modified release pharmaceutical composition for oral administration comprising an active pharmaceutical agent and konjac glucomannan.

In a preferred embodiment of the invention, there is provided konjac glucomannan which is released when the dosage form is tampered with and turns the tampered dosage form into a thick gel mass which slows the absorption of the opioid analgesic such that an abuser is less likely to obtain a rapid "high". In certain preferred embodiments, when the dosage form is tampered with and exposed to a small amount (e. g., less than about 10 ml) of an aqueous liquid (e. g. including but not limited to, water), the dosage form will be unsuitable for injection and/or inhalation.

The term abuse deterrent as used herein is understood to mean that it discourages or is intended to discourage someone from misusing the composition as claimed. In a preferred embodiment of the present invention, the abuse deterrence is understood to be aimed at rendering the solvent extraction and subsequent injection of the active pharmaceutical agent very difficult or impracticable by a potential abuser.

In another embodiment of the present invention, abuse deterrence is also understood to mean that upon inhalation of the crushed composition according to the present invention, the abuser will feel nasal irritation stemming from the incorporation of an irritant in the composition. Upon the addition of the aqueous liquid, the tampered dosage form becomes a thick and viscous gel mass, which makes it unsuitable for injection. Upon oral administration, the tampered dosage form, now in the form of a gel mass, is expected not to "dose dump" the therapeutic agent and thereby, render oral ingestion of the tampered dosage form useless to abusers.

The term "unsuitable for injection" is defined for purposes of the present invention to mean that one would have substantial difficulty manipulating the tampered dosage form with the goal of injecting it with the use of a syringe. The main reasons which would justify a tampered dosage form to be unsuitable for injection are the following: due to pain upon administration or difficulty of pulling the drug into the syringe and/or pushing the tampered dosage form through a syringe. The viscosity of the tampered dosage form thus reduces the potential for abuse of the drug in the dosage form. In some embodiments, konjac glucomannan, is present in an amount as to render solvent evaporation much more difficult. Once the solvent is put in the powdered dose, further heating by abusers to extract the API, will speed up gelation in a media produces a highly viscous gel mass which, in turn, is unsuitable for injection.

The difficulties in the manipulation emanate from the viscosity imparted on the tampered dosage form. This has for effect to reduce the potential for abuse of the opioid analgesic in the dosage form. The gelling agent, konjac glucomannan, may be present in such an amount in the dosage form to prevent the full evaporation of the solvent to an aqueous mixture of the dosage form. This in turn, prevents to concentrate the therapeutic agent, and instead, produces a gel which is unsuitable for injection.

The gelling agent, according to one aspect of the present invention is konjac glucomannan and is released when the dosage form is tampered with and placed in a solvent. This turns the tampered dosage form into a soft gel-like mass which slows the absorption of the opioid analgesic such that an abuser is less unlikely to obtain a rapid "high" since immediate release of the therapeutic agent is avoided. In certain preferred embodiments, when the dosage form is tampered with and exposed to a small amount (e. g., less than about 10 ml) of an liquid media (e. g. , including but not limited to water), the dosage form will be unsuitable for injection and/or inhalation. Upon the addition of the aqueous liquid, the tampered dosage form becomes thick and viscous, rendering it unsuitable for injection and instillation into nostril as well.

According to an embodiment of the present invention, the use of konjac glucomannan has a substantial advantage over what is commonly used as pharmaceutical excipient since it provides abuse deterrence via extraction by solvent over a wide array of solvents while providing modified release properties to the composition. Therefore, according to a preferred embodiment of the present invention, the use of konjac glucomannan is sufficient to provide the modified release profile desired while providing at the same time abuse deterrence via solvent extraction over a wide array of solvents.

Konjac glucomannan can be purchased commercially in average molecular weight ranging from 200,000 to 2,000,000. Acetyl groups found on the backbone of the konjac glucomannan contribute to the solubility of the latter. The acetyl groups and their location is function of the manufacturing process of acetylation of konjac and can be located, on average, at every 9 to 19 sugar units.

There are a number of available konjac gums on the market. The grades vary depending on the glucomannan content and viscosity of the gum. For example, grades of konjac gums are available were the glucomannan content is above 71%, above 74%, above 80%, above 83%, above 86% and above 90%. The viscosities between grades can vary from 6 - 8*10³ mPa·s to 15-18*10³ mPa·s.

In certain embodiments of the present invention, the modified release composition is achieved via a matrix optionally having an additional modified release coating as set forth herein. In certain embodiments of the present invention, a modified release matrix containing another at least one other release modifying agent can yield the desired *in vitro* dissolution rates of the drug(s) releases the opioid analgesic in a pH dependent or pH-independent manner.

The compositions according to the present invention further comprise one or more pharmaceutically acceptable excipients. Such excipients are added to the compositions for a variety of purposes. The pharmaceutically acceptable excipients, that may be present in the compositions according to the present invention, are selected from the group consisting of: diluents, binders, lubricants, disintegrants, glidants, coating agents and release modifying agents.

A non-limiting list of release modifying agents which may be included in a modified release matrix according to the invention includes hydrophilic and/or hydrophobic materials, such as gums, cellulose ethers, acrylic resins, protein derived materials, waxes, shellac, and oils such as hydrogenated castor oil and hydrogenated vegetable oil. However, any pharmaceutically acceptable hydrophobic or hydrophilic sustained-release material which is capable of imparting release modifying properties of the opioid analgesic may be used in accordance with the present invention. Preferred sustained-release polymers include alkylcelluloses such as ethylcellulose, acrylic and methacrylic acid polymers and copolymers; and cellulose ethers, especially hydroxyalkylcelluloses (especially hydroxypropylmethylcellulose) and carboxyalkylcelluloses. Preferred acrylic and methacrylic acid polymers and copolymers include methyl methacrylate, methyl methacrylate copolymers, ethoxyethyl methacrylates, ethyl acrylate, trimethyl ammonioethyl methacrylate, cyanoethyl methacrylate, aminoalkyl methacrylate copolymer, poly (acrylic acid), poly (methacrylic acid), methacrylic acid alkylamine copolymer, poly (methylmethacrylate), poly (methacrylicacid) (anhydride), polymethacrylate, polyacrylamide, poly (methacrylic acid anhydride), and glycidyl methacrylate copolymers. Combinations of two or more of the above materials may be used to obtain the desired dissolution profile.

Some of the preferred pharmaceutically acceptable excipients of the compositions of the present invention include: povidone, sodium hydroxide, isopropyl alcohol, silicified MCC90 (Prosolv®), colloidal silicon dioxide (Aerosil®), dibasic calcium phosphate hydrous, croscarmellose sodium, magnesium oxide heavy, magnesium stearate, microcrystalline cellulose, crospovidone, starches, lactose, iron oxides and and mixtures thereof.

Suitable diluents include for example pharmaceutically acceptable inert fillers such as microcrystalline cellulose, lactose, pregelatinized starch, dibasic calcium phosphate, saccharides, crospovidone, and mixtures of the foregoing.

Suitable binders include, for example, the following: povidone, copovidone, alginic acid, sodium alginate, cellulose derivatives such as hydroxypropyl methylcellulose, hydroxypropyl cellulose, hydroxy ethyl cellulose, methylcellulose, ethyl cellulose, gelatin, starch or starch derivatives and mixtures thereof.

Suitable lubricants include, for example, the following: magnesium-, aluminum- or calcium-stearate, stearic acid, sodium stearyl fumarate, talc, sodium benzoate, glyceryl mono fatty acid, glyceryl monostearate hydrogenated vegetable oil, polyethylene glycol and mixtures thereof.

Suitable disintegrants include, for example, the following: croscarmellose sodium, sodium starch glycolate, maize starch, CMC-Ca, CMC-Na, microcrystalline cellulose, cross-linked PVP, alginic acid, sodium alginate, pregelatinized starch, low-substituted hydroxypropyl cellulose and mixtures thereof.

Suitable anti-adherents include, for example, one or more compounds that are capable of preventing stickiness to surfaces of the punches. Examples of anti-adherents include silicon-containing compounds such as colloidal silicon dioxide, magnesium trisilicate and talc.

The term "treatment" as used herein is understood to mean management and care of a patient or the combating of disease, disorder and/or symptoms. The term "treatment" is meant to be encompassing of the multitude of actions considered by healthcare providers as being part of a subclass of treatment, these include: active treatment; causal treatment; conservative treatment; empiric treatment; expectant treatment; palliative treatment; preventive treatment, prophylactic treatment; rational treatment; specific treatment; and symptomatic treatment.

The abuse-deterrent modified release pharmaceutical composition for oral administration of the present invention can be suitable to use in the treatment of a variety of conditions, but it will typically be used with pain medication to treat, minimize or prevent pain.

The proposed invention would allow one to formulate abuse deterrent compositions containing a drug which is prone to abuse and this is not limited to opioids, it also includes other drugs listed mentioned previously. For exemplary purposes and since opioids are generally the most abused drugs, the present invention allows one skilled in the art to develop abuse deterrent compositions which are equivalent to currently available dosage forms containing the opioid oxycodone provide a range of dosage amounts which includes 5, 10, 15, 20, 30, 40, 60, 80 and 160 mg of oxycodone base per tablet. Currently available dosage forms containing the opioid hydromorphone provide a range of dosage amounts which includes 2, 4, 8, 16, and 32 mg of hydromorphone base per tablet. The proposed invention would allow one to formulate abuse deterrent compositions containing those amounts as well as any other amount within that range or even outside that range so long as such amounts are acceptable to the relevant health authorities.

In light of results obtained, the inventors developed formulations where more than one gelling polymeric compound is present in the abuse-deterrent pharmaceutical compositions. This provided efficient gelling for pharmaceutical compositions in all solvents tested.

The inventors established that gellan gum does not form a gel below pH 2 and in high salt conditions such as 50mM at pH 7.5. Moreover, the inventors determined that konjac glucomannan does not consistently form a strong gel in hydroalcoholic solutions.

The inventors further established that, when combining gellan gum and konjac glucomannan in the right proportions a semi-solid gel was formed in all of the solvents tested. Subsequently, to reduce the amount of konjac glucomannan and gellan used, the inventors established that certain gelling polymeric compounds could yield modified release abuse deterrent pharmaceutical compositions. The at least one gelling polymeric compound is selected from the group consisting of: polysaccharides, sugars, sugar derived alcohols, starches, starch derivatives, cellulose derivatives, carrageenan, pectin, sodium alginate, gellan gum, xanthan gum, poloxamer, carbopol, polyox, povidone, hydroxypropyl methylcellulose (HPMC), hypermellose, and combinations thereof. Preferably, the at least one gelling polymeric compound is selected from the group consisting of: gellan gum, xanthan gum, carrageenan, carbopol, polyox, hydroxypropyl methylcellulose (HPMC), and combinations thereof.

In pharmaceutical composition with abuse deterrent components according to the present invention, the suitable content for konjac glucomannan can range from 3% to 90% w/w (of the total tablet without coating), preferably from 10 % to 80% w/w, more preferably from 30% to 60% w/w, for gellan gum from 1% to 80% w/w (of the total tablet without coating), preferably from 1% to 30% w/w, for a third gelling polymeric compound from 1% to 50% w/w (of the total tablet without coating), preferably from 1% to 30% w/w based on the total weight of the pharmaceutical composition.

A method of manufacturing the pharmaceutical compositions according to the present invention would be through a wet granulation (either aqueous or nonaqueous). This is performed by using a binder solution/suspension or paste of all or part of all or some polymers used in this formulation (or any suitable binders of hydrophilic or hydrophobic gelling or non gelling polymers such as HPMC, PVP, EC, HPC, Polyox®) could be used to make the wet mass of the drug and other excipients in a suitable vessel. The wet mass is then wet milled or directly dried in a suitable vessel. Finally, the dried mass could be reduced into and screened to get suitable mesh sized granules. The granules could be lubricated and punched into tablets with or without additional disintegrants.

Another method of manufacturing the pharmaceutical compositions according to the present invention would be through roller compaction. The ingredients are mixed with a part or all quantity of the lubricant and then are compressed rolled. The compact is then milled into granules which are then lubricated and punched into tablets with or without additional disintegrants.

Yet another method of manufacturing the pharmaceutical compositions according to the present invention would be through powder/granules filled capsules. The final mixture obtained using any one of the previously mentioned methods such as, direct compression, wet granulation or roller compaction is lubricated and filled into suitable size capsule shell with or without additional disintegrants.

Yet another method of manufacturing the pharmaceutical compositions according to the present invention, would be by pelletizing the active pharmaceutical ingredient with suitable excipients and mix the pellets so formed with other excipients to form the matrix. Subsequently, the mixture is either compressed into tablets or filled in capsules as mentioned previously.

Yet another method of manufacturing the pharmaceutical compositions according to the present invention, would be by making immediate release and modified release microparticles containing the active pharmaceutical ingredient and suitable excipients and mixed with other suitable matrix excipients. Subsequently, the mixture is either compressed into tablets or filled in capsules as mentioned previously.

Yet another method of manufacturing the pharmaceutical compositions according to the present invention, would be to manufacture double layered or multilayered tablets by altering the proportion of excipients and punching the blend into distinct layers as part of layered tablets.

### PREFERRED EMBODIMENTS ACCORDING TO THE PRESENT INVENTION

The present invention provides a modified release orally administrable abuse-deterrent pharmaceutical composition comprising: a therapeutically effective amount of an active pharmaceutical ingredient and konjac glucomannan.

The following examples illustrate various aspects of the present invention. They are not to be construed to limit the claims in any manner whatsoever.

### Comparative Example 1

A hydromorphone HCI modified release composition containing konjac glucomannan as abuse deterrent excipient was prepared. The following is a list of the components present in the formulation:

**Table 1 - Components present in composition of Example 1**

| **SN** | **Ingredients** | **(mg)** |
|---|---|---|
| 1 | Hydromorphone HCI | 32 |
| 2 | Konjac glucomannan | 270 |
| 3 | Colloidal silicon dioxide | 2 |
| 4 | Crospovidone XL | 91 |
| 5 | Magnesium stearate | 5 |
| | Total (core) | 400 |
| 6 | Opadry 00H12006 | 20 |
| **Total** | **(core + coating)** | 420 |

### Tablet Preparation

### Preparation of the tablet core

Ingredients 1-4 were mixed in a suitable size polybag and passed through a comill. The resultant blend was mixed in a bin blender for 10 minutes. Finally, magnesium stearate was sieved through a 60 mesh and added to the bin blender and mixed for 3 minutes. The blend was then compressed into tablets.

### Preparation of coated tablets

Opadry® was dissolved in dissolution media, water to get 15% w/v mixture. Core tablets were loaded in a suitable size pan and coated using the above Opadry® mixture.

### Evaluation of dissolution profile

Dissolution profile of the tablets obtained from above mentioned Example 1 was tested in 900 mL dissolution media (Phosphate buffer pH 6.8) using USP1 dissolution apparatus at 100 rpm at 37°C. Samples were taken at 1, 2, 4, 6, 8 and 12 hr and analyzed for the active pharmaceutical ingredient using UV method. The concentration of the drug was calculated using standard curve constructed using pure API and corrected for blank. Sink condition was maintained throughout the test by replacing the equivalent amount of media for each sample taken.

### Evaluation of gelation behaviour

Each coated tablet was crushed in a mortar and pestle to get a fine powder. This was then transferred to 20 mL clear glass vial and 10 mL of solvent was added. It was stirred immediately vigorously and the time taken to get a semi-solid mass that did not fall while inverting the bottle by 180 degree was noted. Gel time was calculated using media at room temperature as well as using boiling media and further boiling the mixture.

### Syringeability and iniectabilitv

In order to abuse the drugs via injection route, abusers typically crush the tablet and dissolve in small amount of water to extract the soluble drug. The ease in the drawing of the mass into the syringe (syringeability) and injection of the mass in the syringe (injectability) was assessed, when possible, using the insulin syringe which they typically use. Crushed tablets in Examples 1 quickly turned into soft semi-solid mass that did not fall upon inversion by 180 degree of the vial within few minutes in the cold medias and within a minute in the hot medias. Due to such soft semi-solid consistency of crushed tablets in those medias (Tables 5 to 8), it was not possible to draw the obtained mass into a syringe for subsequent injection.

### Comparative Example 2

An oxycodone modified release composition containing konjac glucomannan as abuse deterrent excipient was prepared. The following is the list of excipients and their respective amounts:

**Table 2 - Components present in composition of Example 2**

| **SN** | **Ingredients** | **(mg)** |
|---|---|---|
| 1 | Oxycodone HCI | 40 |
| 2 | Konjac glucomannan | 270 |
| 3 | Colloidal silicon dioxide | 3.5 |
| 4 | Crospovidone XL | 180.3 |
| 5 | Magnesium stearate | 6.25 |
| **Total** | **(core)** | 505 |
| 6 | Opadry 00H12006 | 20 |
| **Total** | | 525 |

### Tablet Preparation

### Preparation of the tablet core

Ingredients 1-4 were mixed in a suitable size polybag and passed through a comill. The resultant blend was mixed in a bin blender for 10 minutes. Finally, magnesium stearate was sieved through a 60 mesh and added to the bin blender and mixed for 3 minutes. The blend was then compressed into tablets.

### Preparation of coated tablets

Opadry® was dissolved in DM water to get 15% w/v mixture. The core tablets were loaded in a suitable size pan and coated using the above Opadry® mixture.

### Evaluation of the dissolution profile

The dissolution profile of the tablets obtained from above mentioned Example 2 was studied in 900 mL dissolution media (Phosphate buffer pH 6.8) using USP1 dissolution apparatus at 100 rpm at 37 °C. Samples were taken at 1, 2, 4, 6, 8 and 12 hr and analyzed for the active pharmaceutical ingredient using UV method. The concentration of the drug was calculated using standard curve constructed suing pure API and corrected for blank. Sink condition was maintained throughout the test by replacing the equivalent amount of media for each sample taken.

### Evaluation of gelation behaviour

Each coated tablet obtained from above mentioned Example 2 was crushed in a mortar and pestle to get a fine powder. The powder was then transferred to 20 mL clear glass vial and 10 mL of solvent was added. It was stirred immediately vigorously and the time taken to get a semi-solid mass that did not fall while inverting the bottle at 180 degree was recorded. The gel time was measured using media at room temperature as well as using boiling media and further boiling the mixture.

The powder blend had good flowability and density. The prepared tablet cores were smooth surfaced and with hardness of 13 N and 0% friability. Coating further increased tablet hardness.

Dissolution profile of hydromorphone abuse deterrent tablets mentioned in Example 1 is shown in Table 3 and Figure 1. Dissolution was linear and the percentage of drug release increased with time. Similarly, the dissolution profile of oxycodone abuse deterrent tablets mentioned in Example 2 is shown in Table 4 and Figure 1. Dissolution was linear and the percentage of drug release increased with time.

In order to assess the effectiveness of konjac glucomannan to deter potential abusers from extracting an opioid substance from a modified release composition, gelation tests were carried out to determine the time to gelation of a crushed tablet from Example 1 and Example 2 in 10 ml of media at room temperature (Table 3 and 4 respectively) as well as in boiling media. Time taken to get a semi-solid gel mass that did not fall while inverting the glass vial at 180° was noted. The faster this soft semi-solid mass is formed the chances of drawing the solution and injecting by potential abusers is low. It was noted that the gelation time was within 3 minutes in non-alcoholic medias covering the almost all pH ranges. Since abusers particularly try to dissolve the drug in water, the quick gelation time in water was of added value. Similarly, the tablet formula gelled at 5% ethanol in water. In higher ethanol concentration, a thick liquid viscous fluid mass was obtained. However, in those cases, the viscous fluid mass fell while inverting the glass vial to 180 degree.

As the abusers mostly use heat to extract the drug from the mixture, the effect of heat on gelation were determined by using boiling medias and further boiling the mixture. It was noted that the gelation time decreased from 3 minutes to less than one minute in non-alcoholic medias and 5% ethanol. Similarly, a gel, characterized as not thick, was formed in 10% and 20% ethanol within 4 minutes.

Also, at 40% ethanol concentration a thick liquid viscous fluid mass was obtained. However, the gelled mass fell while inverting the glass vial to 180°.

The dissolution profile for hydromorphone abuse-deterrent modified release tablet (Example 1) were performed in 900 mL dissolution media (Phosphate buffer pH 6.8) using USPI dissolution apparatus at 100 rpm. The results are reported in Table 3 below.

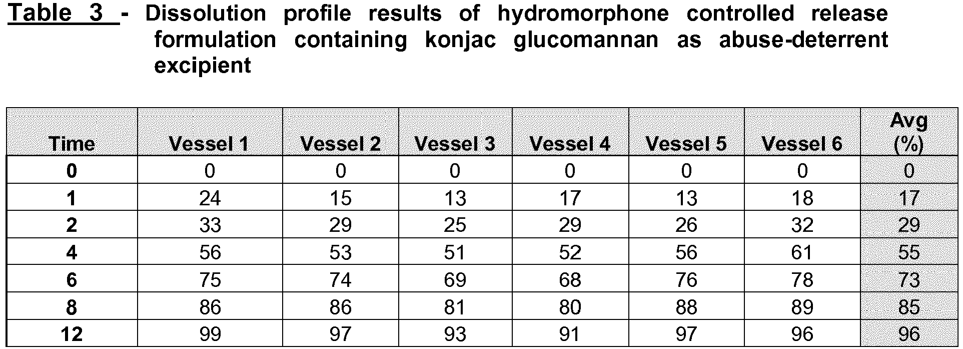

The dissolution profile of oxycodone abuse-deterrent modified release tablet (Example 2) were performed in 900 mL dissolution media (phosphate buffer pH 6.8) using USPI dissolution apparatus at 100 rpm. The results are reported in Table 4 below.

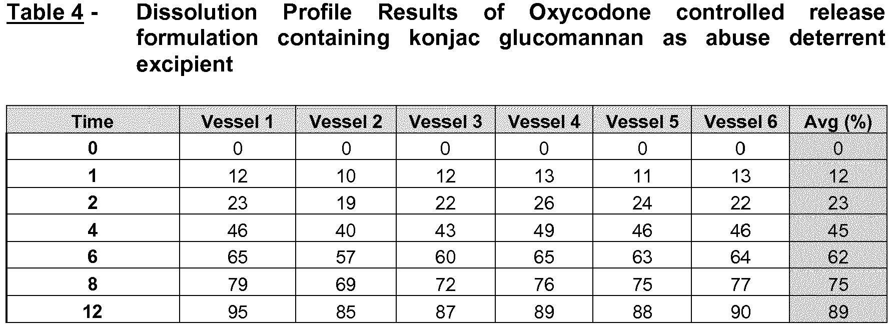

In order to assess the effectiveness of konjac to deter potential abusers from extracting an opioid substance (hydromorphone) from a modified release composition, tests were carried out to determine the time to gelation of a crushed tablet of tablets from Example 1 and Example 2 in 10 ml of various media at room temperature.

**Table 5 - Determination of gelation time of crushed tablet of hydromorphone from Example 1 in 10 ml of media at room temperature**

| **Media** | **Time taken for the gel not to fall when the vial is inverted by 180°** |
|---|---|
| Water | 2.5 min |
| 0.1 N pH 1.1 | 2.5 min |
| Acetate Buffer pH 4 | 2 min |
| Phosphate buffer pH 6.8 | 2 min |
| 0.5% w/v NaOH pH12.5 | 1.8 min |
| 5% v/v ethanol | 7 min |
| 10% v/v ethanol | Viscous dispersion that fell every time while inverting the vial |
| 20% v/v ethanol | Viscous dispersion that fell every time while inverting the vial |
| 40% v/v ethanol | Viscous dispersion that fell every time while inverting the vial |

In order to assess the effectiveness of konjac glucomannan to deter potential abusers from extracting an opioid substance (oxycodone) from a modified release formulation, tests were carried out to determine the time to gelation of a crushed tablet of oxycodone made according to Example 2 in 10 ml of various media at room temperature.

**Table 6 - Determination of gelation time of crushed tablet of oxycodone from Example 2 in 10 ml of media at room temperature**

| **Media** | **Time taken for the gel not to fall when the vial is inverted by 180° (min)** |
|---|---|
| Water | 2 min |
| 0.1 N pH 1.1 | 2 min |
| Acetate Buffer pH 4 | 2 min |
| Phosphate buffer pH 6.8 | 2.5 min |
| 0.5% w/v NaOH pH12.5 | 1.8 min |
| 5% v/v ethanol | 7 min |
| 10% v/v ethanol | Viscous dispersion that fell every time while inverting the vial |
| 20% v/v ethanol | Viscous dispersion that fell every time while inverting the vial |
| 40% v/v ethanol | Viscous dispersion that fell every time while inverting the vial |

In order to assess the effectiveness of konjac to deter potential abusers from extracting an opioid substance (hydromorphone) from a modified release composition, tests were carried out to determine the time to gelation of a crushed tablet of hydromorphone from Example 1 in 10 ml of boiling media.

**Table 7 - Determination of gelation time of crushed tablet of hydromorphone from Example 1 in 10 ml of boiling media**

| **Media** | **Time taken for the gel not to fall when the vial is inverted by 180°** |
|---|---|
| Water | 40 sec |
| 0.1 N pH 1.1 | 40 sec |
| Acetate Buffer pH 4 | 50 sec |
| Phosphate buffer pH 6.8 | 50 sec |
| 0.5% w/v NaOH pH12.5 | 50 sec |
| 5% v/v ethanol | 50 sec |
| 10% v/v ethanol | 3.5 min |
| 20% v/v ethanol | 3.5 min |
| 40% v/v ethanol | Viscous dispersion that fell every time while inverting the vial |

In order to assess the effectiveness of konjac to deter potential abusers from extracting an opioid substance (oxycodone) from a modified release composition, tests were carried out to determine the time to gelation of a crushed tablet of oxycodone from Example 2 in 10 ml of boiling media.

**Table 8 - Determination of gelation time of crushed tablet of Oxycodone from Example 2 in 10 ml of boiling media**

| **Media** | **Time taken for the gel not to fall when the vial is inverted by 180°** |
|---|---|
| Water | 40 sec |
| 0.1 N pH 1.1 | Very thick fluid that did fall very slowly every time while inverting the vial |
| Acetate Buffer pH 4 | 50 sec |
| Phosphate buffer pH 6.8 | 60 sec |
| 0.5% w/v NaOH pH12.5 | 50 sec |
| 5% v/v ethanol | 50 sec |
| 10% v/v ethanol | 3.5 min |
| 20% v/v ethanol | 3.5 min |
| 40% v/v ethanol | Viscous fluid that fell every time while inverting the vial |

### Example 3

A hydromorphone HCI modified release composition was prepared containing konjac glucomannan, gellan gum and xanthan gum as abuse deterrent excipients. It was prepared and tested for abuse deterrence and dissolution behavior. The following is the composition of this example:

**Table 9 - Components present in composition of Example 3**

| **SN** | **Ingredient** | **Qty/Tab** | |
|---|---|---|---|
| | | **(mg)** | **% w/w** |
| 1 | Hydromorphone HCl | 32 | 5.33 |
| 2 | Aerosil | 3 | 0.5 |
| 3 | Magnesium stearate | 6 | 1 |
| 4 | Gellan gum CG HA | 100 | 16.66667 |
| 5 | Konjac glucomannan | 200 | 33.33333 |
| 6 | Xanthan 180 | 50 | 8.333333 |
| 7 | Crospovidone XL | 209 | 34.83 |
| | **Total** | **600** | **100** |

### Method of Manufacture:

A. Pass item 1, 4, 5, 6 and 7 through a 30 mesh and mix those ingredients in a bin blender for 10 minutes.
B. Pass 2 & 3 through a 40 mesh and charge into the bin blender containing the ingredients from Step A.
C. Mix for 3 minutes.

The dissolution of this composition was performed using the USP I basket method. The results of tests on this example are set out in Figure 2.

In order to assess the effectiveness of konjac in the presence of gellan gum and xanthan gum to deter potential abusers from extracting an opioid substance (hydromorphone) from a controlled release composition, tests were carried out to determine the time to gelation of a crushed tablet of hydromorphone HCI from Example 3 in 10 ml in media at room temperature. The results of these tests are set out in Table 10 below.

**Table 10 - Determination of gelation time of crushed tablet of hydromorphone HCI from Example 3 in 10 ml of media at room temperature**

| **Media** | Time **taken for the gel not to fall when the vial is inverted by 180°** |
|---|---|
| Water | 10 sec |
| 0.1 N pH 1.1 | 90 sec |
| Acetate Buffer pH 4 | 10 sec |
| Phosphate buffer pH 7.5 | 20 sec |
| 0.5% w/v NaOH pH12.5 | 15 sec |
| 40% v/v ethanol | 8 min |

### Example 4

A hydromorphone HCI modified release composition was prepared containing konjac glucomannan, gellan gum and HPMC k100M as abuse deterrent excipients. It was prepared and tested for abuse deterrence and dissolution behavior. The following is the composition of this example:

**Table 11 - Components present in composition of Example 4**

| **SN** | **Ingredient** | **Qty/Tab** | |
|---|---|---|---|
| | | **(mg)** | **% w/w** |
| 1 | Hydromorphone HCl | 32 | 5.33 |
| 2 | Aerosil | 3 | 0.5 |
| 3 | Magnesium stearate | 6 | 1 |
| 4 | Gellan gum CG HA | 100 | 16.66667 |
| 5 | Konjac glucomannan | 200 | 33.33333 |
| 6 | HPMC k100M | 50 | 8.333333 |
| 7 | Crospovidone XL | 209 | 34.83 |
| | **Total** | **600** | **100** |

### Method of Manufacture:

A. Pass item 1, 4, 5, 6 and 7 through a 30 mesh and mix those ingredients in a bin blender for 10 minutes.
B. Pass 2 & 3 through a 40 mesh and charge into the bin blender containing the ingredients from Step A.
C. Mix for 3 minutes.

The dissolution of this formulation was performed using the USP I basket method. The results of tests on Example 4 are set out in Figure 2.

In order to assess the effectiveness of konjac in the presence of gellan gum and HPMC to deter potential abusers from extracting an opioid substance (hydromorphone) from a controlled release composition, tests were carried out to determine the time to gelation of a crushed tablet of hydromorphone HCI from Example 4 in 10 ml of dissolution media. The results of these tests are set out in Table 12 below.

**Table 12 - Determination of gelation time of crushed tablet of hydromorphone HCI from Example 4 in 10 ml of media at room temperature**

| **Media** | **Time taken for the gel not to fall when the vial is inverted by 180°** |
|---|---|
| Water | 15 sec - semisolid mass |
| 0.1 N pH 1.1 | 60 sec- semisolid mass |
| Acetate Buffer pH 4 | 20 sec- semisolid mass |
| Phosphate buffer pH 7.5 | 30 sec- semisolid mass |
| 0.5% w/v NaOH pH12.5 | 15 sec-semisolid mass |
| 40% v/v ethanol | 30 sec / was uninjectable/unsyringable viscous mass |

### Example 5

A hydromorphone HCI modified release composition was prepared containing konjac glucomannan, gellan gum and carageenan as abuse deterrent excipients. It was prepared and tested for abuse deterrence and dissolution behavior. The following is the formulation of this example:

**Table 13 - Components present in composition of Example 5**

| **SN** | **Ingredient** | **Qty/Tab** | |
|---|---|---|---|
| | | **(mg)** | **% w/w** |
| 1 | Hydromorphone HCl | 32 | 5.33 |
| 2 | Aerosil | 3 | 0.5 |
| 3 | Magnesium stearate | 6 | 1 |
| 4 | Gellan gum CG HA | 100 | 16.66667 |
| 5 | Konjac glucomannan | 200 | 33.33333 |
| 6 | Carageenan 130 | 50 | 8.333333 |
| 7 | Crospovidone XL | 209 | 34.83 |
| | **Total** | **600** | **100** |

### Method of Manufacture:

A. Pass item 1, 4, 5, 6 and 7 through a 30 mesh and mix those ingredients in a bin blender for 10 minutes.
B. Pass 2 & 3 through a 40 mesh and charge into the bin blender containing the ingredients from Step A.
C. Mix for 3 minutes.

The dissolution of this formulation was performed using the USP I basket method The tests results on Example 5 are set out in Figure 2.

In order to assess the effectiveness of konjac in the presence of gellan gum and carrageenan gum to deter potential abusers from extracting an opioid substance (hydromorphone) from a modified release composition, tests were carried out to determine the time to gelation of a crushed tablet of hydromorphone HCI from Example 5 in 10 ml of media at room temperature. The results of these tests are set out in Table 14 below.

**Table 14 - Determination of gelation time of crushed tablet of hydromorphone HCI from Example 5 in 10 ml of media at room temperature**

| **Media** | **Time taken for the gel not to fall when the vial is inverted by 180°** |
|---|---|
| Water | 15 sec - semisolid mass |
| 0.1 N pH 1.1 | 1 min- semisolid mass |
| Acetate Buffer pH 4 | 30 sec - semisolid mass |
| Phosphate buffer pH 7.5 | 25 sec- semisolid mass |
| 0.5% w/v NaOH pH12.5 | 10 sec - semisolid mass |
| 40% v/v ethanol | 5 min - unsyringable & uninjectable viscous mass |

### Example 6

A hydromorphone HCI modified release composition was prepared containing konjac glucomannan, gellan gum and polyethylene oxide as abuse deterrent excipients. It was prepared and tested for abuse deterrence and dissolution behavior. The following is the formulation of this example:

**Table 15 - Components present in composition of Example 6**

| **SN** | **Ingredient** | **Qty/Tab** | |
|---|---|---|---|
| | | **(mg)** | **%w/w** |
| 1 | Hydromorphone HCl | 32 | 5.33 |
| 2 | Aerosil | 3 | 0.5 |
| 3 | Magnesium stearate | 6 | 1 |
| 4 | Gellan gum CG HA | 100 | 16.66667 |
| 5 | Konjac glucomannan | 200 | 33.33333 |
| 6 | Polyox 303 | 50 | 8.333333 |
| 7 | Crospovidone XL | 209 | 34.83 |
| | **Total** | **600** | **100** |

### Method of Manufacture:

A. Pass item 1, 4, 5, 6 and 7 through a 30 mesh and mix those ingredients in a bin blender for 10 minutes.
B. Pass 2 & 3 through a 40 mesh and charge into the bin blender containing the ingredients from Step A.
C. Mix for 3 minutes.

The dissolution of this composition was performed using the USP I basket method. The test results on Example 6 are set out in Figure 2.

In order to assess the effectiveness of konjac in the presence of gellan gum and polyethylene oxide to deter potential abusers from extracting an opioid substance (hydromorphone) from a modified release composition, tests were carried out to determine the time to gelation of a crushed tablet of hydromorphone HCI from Example 6 in 10 ml of media at room temperature. The results of these tests are set out in Table 16 below.

**Table 16 - Determination of gelation time of crushed tablet of Hydromorphone HCI from Example 6 in 10 ml of media at room temperature**

| **Media** | **Time taken for the gel not to fall when the vial is inverted by 180°** |
|---|---|
| Water | 15 sec - semisolid mass |
| 0.1 N pH 1.1 | 60 sec - semisolid mass |
| Acetate Buffer pH 4 | 30 sec - semisolid mass |
| Phosphate buffer pH 7.5 | 50 sec- semisolid mass |
| 0.5% w/v NaOH pH12.5 | 20 sec- semisolid mass |
| 40% v/v ethanol | Instantly - unsyringable & uninjectable viscous mass |

### Example 7

A hydromorphone HCI modified release composition was prepared containing konjac glucomannan, gellan gum and carbopol as abuse deterrent excipients. It was prepared and tested for abuse deterrence and dissolution behavior. The following is the formulation of Example 7.

**Table 17 - Components present in composition of Example 7**

| **N** | **Ingredient** | **Qty/Tab** | |
|---|---|---|---|
| | | **(mg)** | **%w/w** |
| 1 | Hydromorphone HCl | 32 | 5.33 |
| 2 | Aerosil | 3 | 0.5 |
| 3 | Magnesium stearate | 6 | 1 |
| 4 | Gellan gum CG HA | 100 | 16.66667 |
| 5 | Konjac glucomannan | 200 | 33.33333 |
| 6 | Carbopol 971P | 50 | 8.33333 |
| 7 | Crospovidone XL | 209 | 34.83 |
| | **Total** | **600** | **100** |

### Method of Manufacture:

A. Pass item 1, 4, 5, 6 and 7 through a 30 mesh and mix those ingredients in a bin blender for 10 minutes.
B. Pass 2 & 3 through a 40 mesh and charge into the bin blender containing the ingredients from Step A.
C. Mix for 3 minutes.

The dissolution of this composition was performed using the USP I basket method. The results of tests on Example 7 are set out in Figure 2.

In order to assess the effectiveness of konjac in the presence of gellan gum and carbopol to deter potential abusers from extracting an opioid substance (hydromorphone) from a modified release composition, tests were carried out to determine the time to gelation of a crushed tablet of hydromorphone HCI from Example 7 in 10 ml of media at room temperature. The results of these tests are set out in Table 18 below.

**Table 18 - Determination of gelation time of crushed tablet of hydromorphone HCI from Example 7 in 10 ml of media at room temperature**

| **Media** | **Time taken for the gel not to fall when the vial is inverted by 180°** |
|---|---|
| Water | 15 sec - semisolid mass |
| 0.1 N pH 1.1 | 90 sec- semisolid mass |
| Acetate Buffer pH 4 | 20 sec- semisolid mass |
| Phosphate buffer pH 7.5 | 30 sec- semisolid mass |
| 0.5% w/v NaOH pH12 | 30 sec- semisolid mass |
| 40% v/v ethanol | Instantly- unsyringable & uninjectable viscous mass |

The dissolution profile for hydromorphone HCI abuse-deterrent modified release tablets prepared in Examples 3, 4, 5, 6, and 7 were performed in 900 mL dissolution media (phosphate buffer pH 6.8) using USPI dissolution apparatus at 100 rpm. The results are reported in Table 19 below.

**Table 19 - Dissolution profile results of hydromorphone controlled release formulations from Examples 3, 4, 5, 6 and 7**

| **Time** | **Example 3 (ADTF-166)** | **Example 4 (ADTF-167)** | **Example 5 (ADTF-168)** | **Example 6 (ADTF-169)** | **Example 7 (ADTF-170)** |
|---|---|---|---|---|---|
| 0 | 0 | 0 | 0 | 0 | 0 |
| 60 | 12 | 9 | 14 | 16 | 0 |
| 120 | 18 | 19 | 25 | 27 | 7 |
| 240 | 31 | 36 | 35 | 44 | 20 |
| 360 | 43 | 51 | 48 | 58 | 30 |
| 480 | 53 | 63 | 59 | 69 | 38 |
| 720 | 70 | 80 | 74 | 84 | 51 |

## Claims

1. A modified release orally administrable abuse-deterrent pharmaceutical composition comprising: a therapeutically effective amount of an active pharmaceutical ingredient, konjac glucomannan and gellan gum.

2. A modified release orally administrable abuse-deterrent pharmaceutical composition according to claim 1, wherein the active pharmaceutical ingredient is selected from the group consisting of: opioids and morphine derivatives selected from the group consisting of: oxycodone HCI, hydrocodone bitartrate hydromorphone, oxymorphone, meperidine, propoxyphene, fentanyl, tramadol, codeine, morphine and methadone; antidepressants selected from the group consisting of: barbiturates, benzodiazepines and sleep medications; stimulants selected from the group consisting of: amphetamines and methylphenidate; and dextrometorphan.

3. A modified release orally administrable abuse-deterrent pharmaceutical composition according to any one of claims 1 to 2, wherein said composition provides release of the active pharmaceutical ingredient over 8 hours, over at least 12 hours or over 24 hours.

4. The modified release orally administrable abuse-deterrent pharmaceutical composition according to any one of claims 1 to 3, wherein konjac glucomannan is present in an amount ranging from 3 % to 90 % w/w, from 10 % to 80 % w/w, from 25 % to 65 % w/w, from 30 % to 60 % w/w or from 30 % to 50 % w/w.

5. The modified release orally administrable abuse-deterrent pharmaceutical composition according to any one of claims 1 to 4, wherein the gellan gum is present in an amount ranging from about 1.0% w/w to about 30% w/w.

6. The modified release orally administrable abuse-deterrent pharmaceutical composition according to claim 1 to 5, wherein said composition becomes an uninjectable and unsyringeable gel when tampered and exposed to aqueous, alcoholic, acidic or basic media.

7. The modified release orally administrable abuse-deterrent pharmaceutical composition according to claim 1, further comprising at least one other gelling polymeric compound, wherein the at least one other gelling polymeric compound is selected from the group consisting of: polysaccharides, sugars, sugar derived alcohols, starches, starch derivatives, cellulose derivatives, pectin, sodium alginate, poloxamer, xanthan gum, carrageenan, carbopol, polyethylene oxide, povidone, hydroxypropyl methylcellulose (HPMC) hypermellose and combinations thereof.

8. A modified release orally administrable abuse-deterrent pharmaceutical composition comprising:
- at least one pharmaceutically active ingredient susceptible to abuse;
- konjac glucomannan;
- gellan gum;
- at least one gelling polymeric compound selected from the group consisting of: xanthan gum, polyethylene oxide, carrageenan, carbopol, hydroypropylmethylcellulose and combinations thereof;
- optionally, sodium lauryl sulfate,
- optionally, a nasal irritant, and
- at least one other pharmaceutically acceptable excipient;
wherein said formulation provides release of the active pharmaceutical ingredient and has an in vitro dissolution profile where not more than 60 % of the pharmaceutically active ingredient is dissolved in 6 hours after administration as determined by USP paddles method described in USP XXVI (2003).

9. The modified release orally administrable abuse-deterrent pharmaceutical composition according to claim 8, wherein the gellan gum is present in an amount ranging from 1.0% w/w to 30% w/w.

10. The modified release orally administrable abuse-deterrent pharmaceutical composition according to any one of claims 1 to 9, further comprising at least one nasal irritant selected from the group consisting of: sodium lauryl sulfate, capsaicin and capsaicin analogs selected from resiniferatoxin, tinyatoxin, heptanoylisobutylamide, heptanoyl guaiacylamide, other isobutylamides or guaiacylamides, dihydrocapsaicin, homovanillyl octylester, nonanoyl vanillylamide and mixtures thereof.

11. The modified release orally administrable abuse-deterrent pharmaceutical composition according to claim 10, wherein the nasal irritant is sodium lauryl sulfate and is present in an amount ranging from about 1.0% w/w to about 10% w/w.

12. A modified release orally administrable abuse-deterrent pharmaceutical composition comprising:
a) an active pharmaceutical ingredient susceptible to abuse;
b) konjac glucomannan;
c) sodium lauryl sulfate;
d) gellan gum; and
e) at least one other pharmaceutically acceptable excipient,
for use in the treatment of pain, depressions, anxiety or sleep disorders.

13. A modified release orally administrable abuse-deterrent pharmaceutical composition according to any one of claims 1 to 12 wherein upon tampering and exposure to an aqueous, alcoholic, acidic and/or basic media, said composition becomes an uninjectable and unsyringeable gel.

14. The modified release orally administrable abuse-deterrent pharmaceutical composition according to claims 1 to 13 for use in the treatment of pain or depression.

15. A modified release orally administrable abuse-deterrent pharmaceutical composition for use in the treatment of pain, depression, anxiety or sleep disorders, narcolepsy and Attention-Deficit/Hyperactivity Disorder (ADHD) in human, wherein said composition comprises: a therapeutically effective amount of an active pharmaceutical ingredient susceptible to abuse; konjac glucomannan; gellan gum; optionally, at least one nasal irritant; and at least one other pharmaceutically acceptable excipient.

## Patentansprüche

1. Oral verabreichbare missbrauchssichere pharmazeutische Zusammensetzung mit modifizierter Freisetzung, umfassend: eine therapeutisch wirksame Menge eines pharmazeutischen Wirkstoffs, Konjak Glucomannan und Gellangummi.

2. Oral verabreichbare missbrauchssichere pharmazeutische Zusammensetzung mit modifizierter Freisetzung nach Anspruch 1, wobei der pharmazeutische Wirkstoff ausgewählt ist aus der Gruppe bestehend aus:
Opioiden und Morphinderivaten, ausgewählt aus der Gruppe bestehend aus:
Oxycodon-HCl, Hydrocodonbitartrat-Hydromorphon, Oxymorphon, Meperidin, Propoxyphen, Fentanyl, Tramadol, Codein, Morphin und Methadon;
Antidepressiva, ausgewählt aus der Gruppe bestehend aus:
Barbituraten, Benzodiazepinen und Schlafmitteln;
Stimulantien, ausgewählt aus der Gruppe bestehend aus: Amphetaminen und Methylphenidat;
und Dextrometorphan.

3. Oral verabreichbare missbrauchssichere pharmazeutische Zusammensetzung mit modifizierter Freisetzung nach einem der Ansprüche 1 bis 2, wobei die Zusammensetzung eine Freisetzung des pharmazeutischen Wirkstoffs für 8 Stunden, für mindestens 12 Stunden oder für 24 Stunden bereitstellt.

4. Oral verabreichbare missbrauchssichere pharmazeutische Zusammensetzung mit modifizierter Freisetzung nach einem der Ansprüche 1 bis 3, wobei Konjak Glucomannan in einer Menge vorliegt, die von 3 Gew.-% bis 90 Gew.-%, von 10 Gew.-% bis 80 Gew.-%, von 25 Gew.-% bis 65 Gew.-%, von 30 Gew.-% bis 60 Gew.-% oder von 30 Gew.-% bis 50 Gew.-% reicht.

5. Oral verabreichbare missbrauchssichere pharmazeutische Zusammensetzung mit modifizierter Freisetzung nach einem der Ansprüche 1 bis 4, wobei der Gellangummi in einer Menge vorliegt, die von ungefähr 1,0 Gew.-% bis ungefähr 30 Gew.-% reicht.

6. Oral verabreichbare missbrauchssichere pharmazeutische Zusammensetzung mit modifizierter Freisetzung nach Anspruch 1 bis 5, wobei die Zusammensetzung zu einem nicht injizierbaren und nicht spritzbaren Gel wird, wenn sie manipuliert und wässrigen, alkoholischen, sauren oder basischen Medien ausgesetzt wird.

7. Oral verabreichbare missbrauchssichere pharmazeutische Zusammensetzung mit modifizierter Freisetzung nach Anspruch 1, ferner umfassend mindestens eine andere gelierende Polymerverbindung, wobei die mindestens eine andere gelierende Polymerverbindung ausgewählt ist aus der Gruppe bestehend aus:
Polysacchariden, Zuckern, von Zucker abgeleiteten Alkoholen, Stärken, Stärkederivaten, Cellulosederivaten, Pektin, Natriumalginat, Poloxamer, Xanthangummi, Carrageenan, Carbopol, Polyethylenoxid, Povidon, Hydroxypropylmethylcellulose (HPMC) Hypermellose und Kombinationen davon.

8. Oral verabreichbare missbrauchssichere pharmazeutische Zusammensetzung mit modifizierter Freisetzung, umfassend:
- mindestens einen pharmazeutischen Wirkstoff, der gegenüber einem Missbrauch anfällig ist;
- Konjak Glucomannan;
- Gellangummi;
- mindestens eine gelierende Polymerverbindung, ausgewählt aus der Gruppe bestehend
aus:
Xanthangummi, Polyethylenoxid, Carrageenan, Carbopol, Hydroxypropylmethylcellulose und Kombinationen davon;
- gegebenenfalls Natriumlaurylsulfat,
- gegebenenfalls ein Nasenreizmittel und
- mindestens einen anderen pharmazeutisch unbedenklichen Hilfsstoff;
wobei die Formulierung eine Freisetzung des pharmazeutischen Wirkstoffs bereitstellt und ein In-vitro-Auflösungsprofil aufweist, bei dem nicht mehr als 60 % des pharmazeutischen Wirkstoffs in 6 Stunden nach der Verabreichung gelöst werden, wie durch das USP-Paddelverfahren bestimmt, das in USP XXVI (2003) beschrieben ist.

9. Oral verabreichbare missbrauchssichere pharmazeutische Zusammensetzung mit modifizierter Freisetzung nach Anspruch 8, wobei der Gellangummi in einer Menge vorliegt, die von 1,0 Gew.-% bis 30 Gew.-% reicht.

10. Oral verabreichbare missbrauchssichere pharmazeutische Zusammensetzung mit modifizierter Freisetzung nach einem der Ansprüche 1 bis 9, ferner umfassend mindestens ein Nasenreizmittel, ausgewählt aus der Gruppe bestehend aus:
Natriumlaurylsulfat, Capsaicin und Capsaicin-Analoga, ausgewählt aus Resiniferatoxin, Tinyatoxin, Heptanoylisobutylamid, Heptanoylguaiacylamid, anderen Isobutylamiden oder Guaiacylamiden, Dihydrocapsaicin, Homovanillyloctylester, Nonanoylvanillylamid und Gemischen davon.

11. Oral verabreichbare missbrauchssichere pharmazeutische Zusammensetzung mit modifizierter Freisetzung nach Anspruch 10, wobei das Nasenreizmittel Natriumlaurylsulfat ist und in einer Menge vorliegt, die von ungefähr 1,0 Gew.-% bis ungefähr 10 Gew.-% reicht.

12. Oral verabreichbare missbrauchssichere pharmazeutische Zusammensetzung mit modifizierter Freisetzung, umfassend:
a) einen pharmazeutischen Wirkstoff, der gegenüber einem Missbrauch anfällig ist;
b) Konjak Glucomannan;
c) Natriumlaurylsulfat;
d) Gellangummi; und
e) mindestens einen anderen pharmazeutisch unbedenklichen Hilfsstoff,
zur Verwendung bei der Behandlung von Schmerzen, Depressionen, Angst- oder Schlafstörungen.

13. Oral verabreichbare missbrauchssichere pharmazeutische Zusammensetzung mit modifizierter Freisetzung nach einem der Ansprüche 1 bis 12, wobei die Zusammensetzung nach der Manipulation und Exposition gegenüber wässrigen, alkoholischen, sauren und/oder basischen Medien zu einem nicht injizierbaren und nicht spritzbaren Gel wird.

14. Oral verabreichbare missbrauchssichere pharmazeutische Zusammensetzung mit modifizierter Freisetzung nach den Ansprüchen 1 bis 13 zur Verwendung bei der Behandlung von Schmerzen oder Depressionen.

15. Oral verabreichbare missbrauchssichere pharmazeutische Zusammensetzung mit modifizierter Freisetzung zur Verwendung bei der Behandlung von Schmerzen, Depressionen, Angst- oder Schlafstörungen, Narkolepsie und einer Aufmerksamkeitsdefizit-Hyperaktivitätsstörung (ADHS) beim Menschen, wobei die Zusammensetzung Folgendes umfasst:
eine therapeutisch wirksame Menge eines pharmazeutischen Wirkstoffs, der gegenüber einem Missbrauch anfällig ist;
Konjak Glucomannan;
Gellangummi;
gegebenenfalls mindestens ein Nasenreizmittel;
und mindestens einen anderen pharmazeutisch unbedenklichen Hilfsstoff.

## Revendications

1. Composition pharmaceutique de dissuasion d'abus administrable par voie orale à libération modifiée comprenant : une quantité thérapeutiquement efficace d'un principe pharmaceutique actif, du konjac glucomannan et de la gomme gellane.

2. Composition pharmaceutique de dissuasion d'abus administrable par voie orale à libération modifiée selon la revendication 1, ledit principe pharmaceutique actif étant choisi dans le groupe constitué par : les opioïdes et les dérivés de morphine choisis dans le groupe constitué par : le HCl d'oxycodone, le bitartrate d'hydrocodone, l'hydromorphone, l'oxymorphone, la mépéridine, le propoxyphène, le fentanyl, le tramadol, la codéine, la morphine et la méthadone ; les antidépresseurs choisis dans le groupe constitué par : les barbiturates, les benzodiazépines et les somnifères ; les stimulants choisis dans le groupe constitué par : les amphétamines et le méthylphénidate ; et le dextrométorphane.

3. Composition pharmaceutique de dissuasion d'abus administrable par voie orale à libération modifiée selon l'une quelconque des revendications 1 à 2, ladite composition assurant la libération du principe pharmaceutique actif durant 8 heures, durant au moins 12 heures ou durant 24 heures.

4. Composition pharmaceutique de dissuasion d'abus administrable par voie orale à libération modifiée selon l'une quelconque des revendications 1 à 3, ledit konjac glucomannan étant présent en une quantité allant de 3 % à 90 % en p/p, de 10 % à 80 % en p/p, de 25 % à 65 % en p/p, de 30 % à 65 % en p/p ou de 30 % à 50 % en p/p.

5. Composition pharmaceutique de dissuasion d'abus administrable par voie orale à libération modifiée selon l'une quelconque des revendications 1 à 4, ladite gomme gellane étant présente en une quantité allant d'environ 1 % en p/p à environ 30 % en p/p.

6. Composition pharmaceutique de dissuasion d'abus administrable par voie orale à libération modifiée selon l'une quelconque des revendications 1 à 5, ladite composition devenant un gel non prélevable par seringue et non injectable lorsqu'elle est altérée et exposée à un milieu aqueux, alcoolique, acide ou basique.

7. Composition pharmaceutique de dissuasion d'abus administrable par voie orale à libération modifiée selon la revendication 1, comprenant en outre au moins un autre composé polymère gélifiant, ledit au moins un autre composé polymère gélifiant étant choisi dans le groupe constitué par : les polysaccharides, les sucres, les alcools dérivés de sucres, les amidons, les dérivés d'amidon, les dérivés de cellulose, la pectine, l'alginate de sodium, un poloxamère, la gomme xanthane, la carraghénane, le carbopol, l'oxyde de polyéthylène, la povidone, l'hydroxypropylméthylcellulose (HPMC), l'hypermellose et des combinaisons de ceux-ci.

8. Composition pharmaceutique de dissuasion d'abus administrable par voie orale à libération modifiée comprenant :
- au moins un principe pharmaceutique actif susceptible d'abus ;
- du konjac glucomannan ;
- de la gomme gellane ;
- au moins un composé polymère gélifiant choisi dans le groupe constitué par : la gomme xanthane, l'oxyde de polyéthylène, la carraghénane, le carbopol, l'hydroypropylméthylcellulose et des combinaisons de ceux-ci ;
- éventuellement, du laurylsulfate de sodium,
- éventuellement, un irritant nasal, et
- au moins un autre excipient pharmaceutiquement acceptable ;
ladite formulation assurant la libération du principe pharmaceutique actif et présentant un profil de dissolution in vitro où pas plus de 60 % du principe pharmaceutique actif sont dissous dans les 6 heures après administration comme le détermine la méthode des paddles de la pharmacopée américaine (USP) décrite dans USP XXVI (2003).

9. Composition pharmaceutique de dissuasion d'abus administrable par voie orale à libération modifiée selon la revendication 8, ladite gomme gellane étant présente en une quantité allant de 1,0 % en p/p à 30 % en p/p.

10. Composition pharmaceutique de dissuasion d'abus administrable par voie orale à libération modifiée selon l'une quelconque des revendications 1 à 9, comprenant en outre au moins un irritant nasal choisi dans le groupe constitué par : le laurylsulfate de sodium, la capsaïcine et les analogues de capsaïcine choisis parmi la résinifératoxine, la tinyatoxine, l'heptanoylisobutylamide, le guaïacylamide d'heptanoyle, les autres isobutylamides ou guaïacylamides, la dihydrocapsaïcine, l'octylester d'homovanillyle, le vanillylamide de nonanoyle et des mélanges de ceux-ci.

11. Composition pharmaceutique de dissuasion d'abus administrable par voie orale à libération modifiée selon la revendication 10, ledit irritant nasal étant le laurylsulfate de sodium et étant présent en une quantité allant d'environ 1,0 % en p/p à environ 10 % en p/p.

12. Composition pharmaceutique de dissuasion d'abus administrable par voie orale à libération modifiée comprenant :
a) un principe pharmaceutique actif susceptible d'abus ;
b) du konjac glucomannan ;
c) du laurylsulfate de sodium ;
d) de la gomme gellane ; et
e) au moins un autre excipient pharmaceutiquement acceptable,
pour utilisation dans le traitement de la douleur, des dépressions, de l'anxiété ou des troubles du sommeil.

13. Composition pharmaceutique de dissuasion d'abus administrable par voie orale à libération modifiée selon l'une quelconque des revendications 1 à 12, lors de l'altération et de l'exposition à un milieu aqueux, alcoolique, acide et/ou basique, ladite composition devenant un gel non prélevable par seringue et non injectable.

14. Composition pharmaceutique de dissuasion d'abus administrable par voie orale à libération modifiée selon l'une quelconque des revendications 1 à 13 pour utilisation dans le traitement de la douleur ou de la dépression.

15. Composition pharmaceutique de dissuasion d'abus administrable par voie orale à libération modifiée pour utilisation dans le traitement de la douleur, de la dépression, de l'anxiété ou des troubles du sommeil, de la narcolepsie et du trouble du déficit de l'attention avec hyperactivité (TDAH) chez l'homme, ladite composition comprenant : une quantité thérapeutiquement efficace d'un principe pharmaceutique actif susceptible d'abus ; du konjac glucomannan ; de la gomme gellane ; éventuellement, au moins un irritant nasal ; et au moins un autre excipient pharmaceutiquement acceptable.
